# EUROPEAN PATENT APPLICATION

(11) **EP 3 228 694 A1**
(43) Date of publication of application: **11.10.2017**
(21) Application number: 16163707.9
(22) Date of filing: 04.04.2016
(51) Int. Cl.: C12M 1/22, C12M 3/00, C12M 1/00

(54) **INPUT/OUTPUT SYSTEM FOR AUTOMATICALLY SUPPLYING PETRI DISHES TO A PROCESSING STAGE AND A CARRIER FOR MANUALLY SUPPLYING PETRI DISHES TO AN INPUT/OUTPUT SYSTEM**

(71) Applicant: Clever Culture Systems AG, 8088 Bäch (CH)
(72) Inventor: Böhm, Alexander, 9020 Klagenfurt am Wörthersee (AT); Bogilovic, Nedim, 9020 Klagenfurt am Wörthersee (AT); Stiegmaier, Wolfgang, 8740 Zeltweg (AT)
(74) Representative: Schwarz & Partner Patentanwälte OG

(57) **Abstract**

An input/output system (1) for automatically supplying petri dishes (16) to a processing stage in a petri dishes processing apparatus (34) with an endless conveyor (2), a plurality of racks (6), which are arranged on the endless conveyor (2) and which are configured to hold a plurality of petri dishes (16), and an elevator (13) configured to transfer the petri dishes (16) from the racks (6) to a conveyor platform (5) on after the other. Furthermore, the input/output system (1) comprises a petri dish input section (21) located on the endless conveyor (2). The petri dish input section (21) is configured to receive at least one stack of petri dishes (16) stored in a carrier (26) and to transfer it into at least one rack (6) position at the petri dish input section (21).

## Description

The present invention is related to an input/output system for automatically supplying petri dishes to a processing stage in a petri dishes processing apparatus, comprising:
a plurality of racks displaceably arranged in an orbit path, each rack being configured to store a plurality of petri dishes in a vertically stacked manner, and an elevator arranged at a hand-over position at the orbit path, wherein the elevator is configured to lift a stack of petri dishes contained in one of said racks, while said rack is positioned at the hand-over position.

Furthermore the present invention is related to a carrier for manually supplying petri dishes to an input/output system of a petri dishes processing apparatus.

Carriers providing the ability to carry a high amount of petri dishes from one place to another place and to manually transfer petri dishes to an input/output system of a petri dishes processing apparatus are known in the art. Such carriers comprise a plurality of compartments as first in last out reservoirs for storing petri dishes in a stacked manner. Problems with these carriers are that the carriers need to be loaded and unloaded with petri dishes by hand, wherein the petri dishes are transferred into and out of the carrier, respectively, individually or in piles of petri dishes. This not only constitutes a safety risk for the users working with the carriers, there is also the possibility that probes stored in the petri dishes are damaged, especially due to a lack of attention in the process of handling the petri dishes by hand.

Furthermore input/output systems providing the ability to automatically supply dishes to a further processing stage are known in the art. The patent application EP 2 482 079 A2 discloses such an input/output system for storing and handling petri dishes and providing and supplying the petri dishes to a processing stage for optically capturing the probes stored in the petri dishes. The device comprises a storage system with a rectangular shaped housing and an elevator configured as a transfer device to transfer petri dishes between the storage system and the processing stage. The elevator is situated within the rectangular shaped housing. The storage system comprises two independently rotatable carrousels, wherein one of the two carrousels is of a hollow cylindrical shape and the other one is of a cylindrical shape. The cylindrical shaped carrousel is situated within the hollow cylindrical shaped carrousel in order to increase the amount of stored petri dishes in the storage system. Each carrousel holds a plurality of racks, wherein each rack has a plurality of pockets configured to hold an individual petri dish. For outputting and inputting petri dishes into the cylindrical shaped carrousel the hollow cylindrical shaped carrousel has a gap, through which the elevator can access the petri dishes stored in the cylindrical shaped carrousel. The elevator is disposed in an edge of the housing and comprises a trolley, which trolley can be displaced in a vertical direction and which trolley has a shovel rotatably mounted about a vertical axes on the trolley. The elevator is configured to individually supply petri dishes from the storage system to the processing stage and receive petri dishes from the processing stage and store them in the storage system. The processing stage is configured to open the petri dishes for optically capturing the probes stored in the petri dishes. Moreover in an optional embodiment the input/output system known from the patent application EP 2 482 079 A2 can be equipped with a feed system for automatically supplying petri dishes from a laboratory environment to the storage system and discharge petri dishes from the storage system to the laboratory environment.

The downside of the input/output system disclosed in the patent application EP 2 482 079 A2 is that the storage system needs to be loaded with petri dishes and emptied out by hand. Also the optional available feed system for automatically supplying petri dishes to the storage system and discharge petri dishes from the storage system does not remedy this situation, since the feed system also needs to be fed with individual petri dishes by hand and since the outputted petri dishes need to be led away by hand. As a result loading the storage system and unloading the storage system is very time-consuming. Moreover, due to handling each petri dish individually by hand the risk of damaging the probes stored inside the petri dishes is very high. In addition, the known system has the disadvantage that the storage system is very space consuming due to its circular construction, although the storage system is built by two carrousels. Due to the circular shape of the carrousels a majority of the space inside the housing is not used, which not only reduces the amount of petri dishes that can be stored in the storage device, this results also in a higher energy demand for air conditioning if the storage system is for example implemented in an incubator. Furthermore, since one carrousel is encapsulated in the other one the accessibility of the petri dishes stored in the encapsulated carrousel is very poor and requires because of that a lot of time. This is even more aggravated due to the fact that every petri dish is supplied to the storage and discharged from the storage system by the same elevators.

It is an objective of the present invention to provide a compact input/output system that avoids the drawbacks of the known system and that increase the transfer rate of petri dishes and increases the safety in handling the petri dishes.

This objective is achieved with a system wherein the plurality of racks are arranged on an endless conveyor, which endless conveyor is configured to move the racks along the orbit path and to supply one rack after the other to the hand-over position, the elevator is configured to stepwise lift the stack of petri dishes contained in the rack at the hand-over position by the height of the uppermost petri dish of the stack of petri dishes and to repeat said lifting operation for each petri dish of the stack of petri dishes, such that the uppermost petri dish is presented to a receiving position on a conveyor platform, wherein a pusher is displaceably mounted at the conveyor platform and is configured to receive the petri dish at the receiving position and to move it to the processing stage.

Furthermore this objective is achieved with a carrier wherein the carrier comprises:
a body with at least one compartment, preferably with a plurality of compartments, configured to hold a plurality of petri dishes in a stacked manner, which at least one compartment is at least partially defined by sidewalls and open at an upper side of the body and at a bottom of the body, and a mechanism for releasing petri dishes, which mechanism comprises a releasing element and at least one retaining element, which at least one retaining element is mechanically connected to the one releasing element and displaceably, preferably rotatably, mounted at the bottom of the body, wherein by operating the at least one releasing element the retaining element is displaceable between a locking position and a releasing position, wherein in the locking position the retaining element holds the petri dishes inside the compartments and wherein in the releasing position the retaining element releases the petri dishes for being discharged through the bottom.

Due to the fact, that the input/output system according to the invention is just used for feeding petri dishes to the processing stage, the input/output system enables the supply of petri dishes to the processing stage of the petri dishes processing apparatus in a much faster way.

Furthermore, the configuration of the elevator according to the present invention advantageously enhances the transfer rate of petri dishes from the endless conveyor to the receiving position due to the fact that the elevator manipulates a plurality of petri dishes in one movement and does not move petri dishes individually such as the system known from the prior art. Although a plurality of petri dishes are moved in one movement the elevator supplies the petri dishes one after the other to the receiving position, where they are fed to the processing stage attached to the input/output system by the pusher.

With the carrier according to the present invention a plurality of petri dishes can be easily and safely released by just operating the releasing element, which releasing element is mechanically connected to the at least one retaining element. By operating the releasing element the at least one retaining element is displaced from the locking position to the releasing position or vice versa, whereby the petri dishes stored in the carrier are held inside the compartments or released through the bottom of the body.

Due to the advantageous construction of the mechanism and due to the fact, that the petri dishes are stored in a stacked manner inside the at least one compartment of the carrier the petri dishes can be supplied to the input/output system by just placing the carrier on the endless conveyor. By placing the carrier on the endless conveyor the stack of petri dishes stored in the at least one compartment of the body of the carrier is merged into the a rack disposed on the endless conveyor. Due to that there is no need to handle the petri dishes individually by hand, whereby the risk of damaging probes stored inside the petri dishes is a lot lower.

In a preferred embodiment of the invention each rack comprises a base for supporting the stack of petri dishes and a plurality of poles mounted on the base and extending essentially parallel to a lifting axis of the elevator, wherein the poles are configured to encompass the stack of petri dishes. Preferably the base is built as a U-shaped element. This has the advantage that petri dishes contained in the rack at the hand-over position are easy to access by the elevator.

Advantageously, the endless conveyor is configured as a chain conveyer comprising a chain, which chain is driven by a first motor, wherein the bases of the racks are mounted on the chain, preferably at an equal distance to each other. This has the advantage that the endless conveyor can be configured as an elongated endless conveyor. Due to the elongated shape the endless conveyor can store a high amount of petri dishes in a very small footprint, which allows to build the input/output system more compact compared to the systems known from the prior art. Due to the elongated shape several input/output systems can be arranged side by side without any major change in construction or in operation of the petri dish processing apparatus, whereby the supply of petri dishes to the processing stage in the petri dishes processing apparatus can be increased.

In a preferred embodiment the input/output system comprises eight racks, wherein each rack is configured to hold twenty petri dishes at maximum. In another embodiment each rack is configured to hold more than twenty petri dishes.

In an advantageous embodiment of the input/output system, the input/output system comprises a first sensor and a second sensor. The first sensor is configured to monitor the filling level of petri dishes of the rack while the rack is positioned at the hand-over position. In another embodiment the first sensor is simply configured to monitor if the rack positioned at the hand-over position is filled with petri dishes or if the rack positioned at the hand-over position is empty. The second sensor is configured to monitor a presence of a petri dish at the receiving position on the conveyer platform. In a preferred embodiment the second sensor additionally monitors the shape of the petri dish including the height of the petri dish. Advantageously, the first sensor and/or the second sensor is/are designed as an optical sensor, an RFID sensors, an electromagnetic sensor, a contact sensor and/or a weight sensor and the first sensor and the second sensor are connected to a control unit, which control unit controls the first motor driving the chain and a drive of the elevator. This has the advantage that the movement of the chain and the movement of the elevator can be adjusted in accordance with the amount of petri dishes contained in the rack at the hand-over position and to the height and the shape of the petri dishes contained in the rack, respectively.

In a preferred embodiment the endless conveyer comprises a petri dish input section being configured to receive at least one stack of petri dishes and to transfer it into at least one rack positioned at the petri dish input section. Advantageously, the petri dish input section is defined by a plurality of the racks, preferably by four racks, aligned at equal distances from each other, wherein preferably the petri dish input section is located at a U-turn of the endless conveyor. This has the advantage that the input/output system is easy to access for loading petri dishes into the racks situated in the petri dish input section. Furthermore, this has the advantage that for loading petri dishes into the endless conveyor the alignment of the racks is always the same, whereby the racks in the loading position can be loaded with a carrier holding a plurality of stacks of petri dishes. As a result a big amount of petri dishes can be loaded into the endless conveyor at once.

In another preferred embodiment the input/output system comprises third sensors configured to monitor the filling level of racks located at the petri dish input section. Advantageously, the third sensors are connected to the control device and are designed as optical sensors, electromagnetic sensors, contact sensors and/or weight sensors. This has the advantage that the control unit can monitor if the racks located at the petri dish input section are empty and that the control unit can signal that the input/output system is ready to be loaded with another batch of petri dishes. In another more simple embodiment the third sensors do not monitor the filling level of the racks located at the petri dish input section, they just monitor the presence of a petri dish at the very bottom of the racks.

In a preferred embodiment the carrier comprises a handle, which handle is retractably mounted in or on the body on the upper side of the body. Preferably, the handle is retractably mounted in such a way in or on the body of the carrier that the handle does not essentially retract from an area configured at the upper side of the body in a retraction position. In a carrying position the handle protrudes from the area configured at the upper side of the body. This has the advantage that the carrier can be easily picked up by the handle and that the carrier can be easily transported by a person, e.g. laboratory stuff. Advantageously, the handle falls back into the retraction position by its own weight when the carrier is put down, whereby no further movement for retracting the handle is necessary.

In a further preferable embodiment the handle is the releasing element, wherein by rotating the handle in an opening direction the retaining element is displaced from its locking position into its releasing position and wherein by rotating the handle in a locking direction the retaining element is displaced from its releasing position into its locking position. This has the advantage that there is no need for further elements to release the petri dishes stored in the carrier. Furthermore the release of petri dishes is easy to handle. Advantageously, the body comprises stops and markings which indicate the position of the handle and retaining element.

In another preferable embodiment the carrier is attachable to the input/output system and each compartment comprises recesses in its sidewall, which recesses are configured to receive guide elements arranged at the input/output system, when the carrier is attached to the input/output system. Advantageously, the poles of the stacks are configured as the guiding elements. This has the advantage that supplementary elements can be avoided and that the carrier is attached to the input/output system in the right manner.

In another preferable embodiment the releasing element is configured by at least one engagement element, which at least one engagement element is connected to the at least one retaining element and which at least one engagement element is configured for a positive engagement with a releasing device of the input/output system, when the carrier is attached to the input/output system. Preferably, the releasing device of the input/output system is connected to the control unit. This has the advantage that the release of the petri dishes into the racks of the endless conveyor can be controlled by the control unit of the input/output system.

In another preferable embodiment the mechanism comprises two retaining elements independently pivotally mounted at the bottom of the body, wherein one of the two retaining elements is non-rotatably connected to the engagement element. Furthermore the mechanism comprises at least one gear wheel, which mechanically connects the two retaining elements, wherein by rotating the engagement element through the releasing device the two retaining elements are rotated in the opposite direction to each other. This has the advantage that the petri dishes can be released into the racks of the endless conveyor in a very smooth way.

Advantageously, the body is made of Polycarbonate, Acrylnitril-Butadien-Styrol or a blend of Acrylnitril-Butadien-Styrol, e.g. Makrolon.

In another preferable embodiment the body of the carrier is designed in such a manner, that the carrier is configured to be stackable with another carrier. This has the advantage that the carriers can be stored with a very small footprint and are not in anyone's way if they are not in use.

These and further advantageous embodiments of the invention will be explained based on the following description and the accompanying drawings. The person skilled in the art will understand that various embodiments may be combined.
Figure 1 shows an embodiment of the input/output system according to the present invention in a perspective view.
Figure 2 shows the embodiment of the input/output system according to figure 1 in a side view.
Figure 3 shows the embodiment of the input/output system according to figure 1 in a front view.
Figure 4 shows the embodiment of the input/output system according to figure 1 in a top view.
Figure 5 shows an embodiment of a carrier according to the present invention in a view from below, wherein the carrier is loaded with four stacks of petri dishes.
Figure 6 shows the embodiment of the carrier according to figure 5 in a view from below, wherein the carrier is empty.
Figure 7 shows the embodiment of the carrier according to figure 5 in a perspective view.
Figure 8 shows the embodiment of the input/output system according to figure 1 and the embodiment of the carrier according to figure 5 in the moment of loading the input/output system with petri dishes stored in the carrier in a perspective view.
Figures 9 shows the embodiment of the input/output system according to figure 1 and the embodiment of the carrier according to figure 5 with a partially loaded endless conveyor in a perspective view.
Figure 10 shows the embodiment of the input/output system according to figure 1 and the embodiment of the carrier according to figure 5 in the moment of lifting petri dishes one by the other to a conveyor platform in a perspective view.
Figure 11 shows the embodiment of the input/output system according to figure 1 and the embodiment of the carrier according to figure 5 with an emptied out endless conveyor in a perspective view.
Figures 12 and 13 respectively show the moment of loading the conveyor system of the input/output system according to figure 1 with the carrier according to figure 5 in a more detail view.
Figure 14 shows an embodiment of a petri dishes processing apparatus with two input/output systems according to figure 1 and a plurality of carriers according to figure 5 in a perspective view.

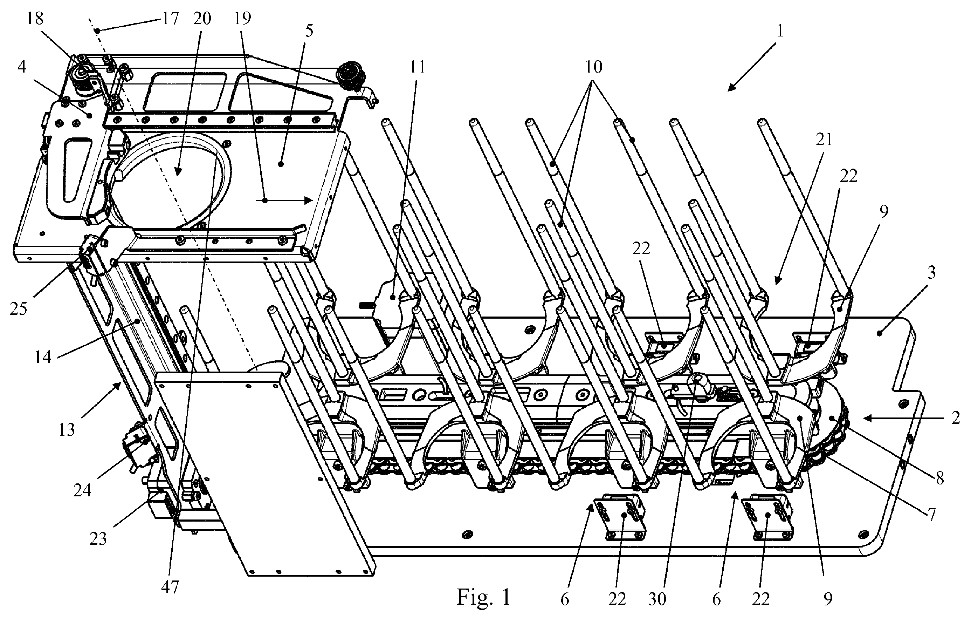

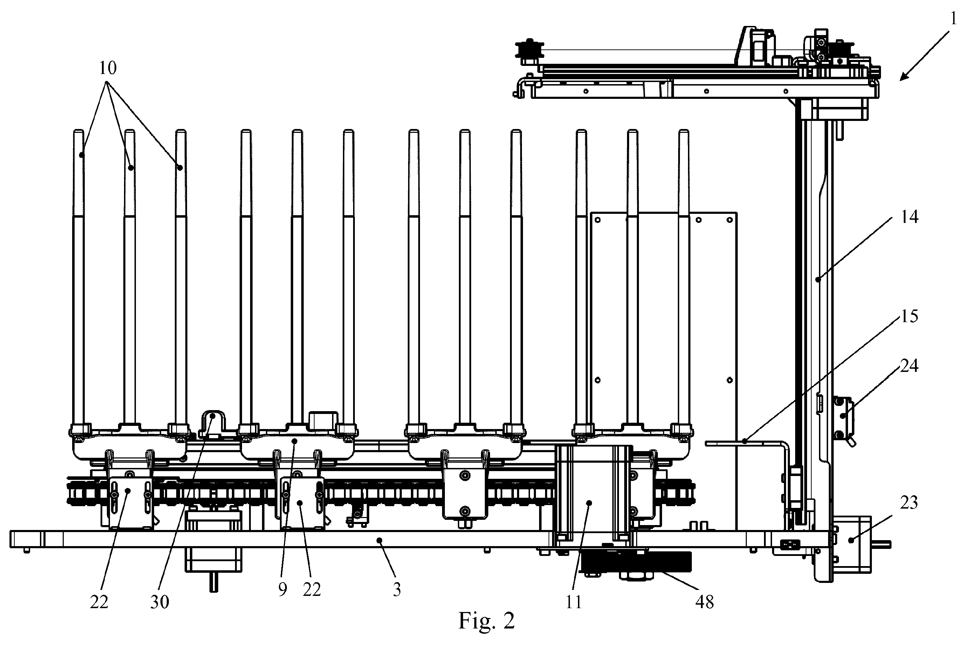

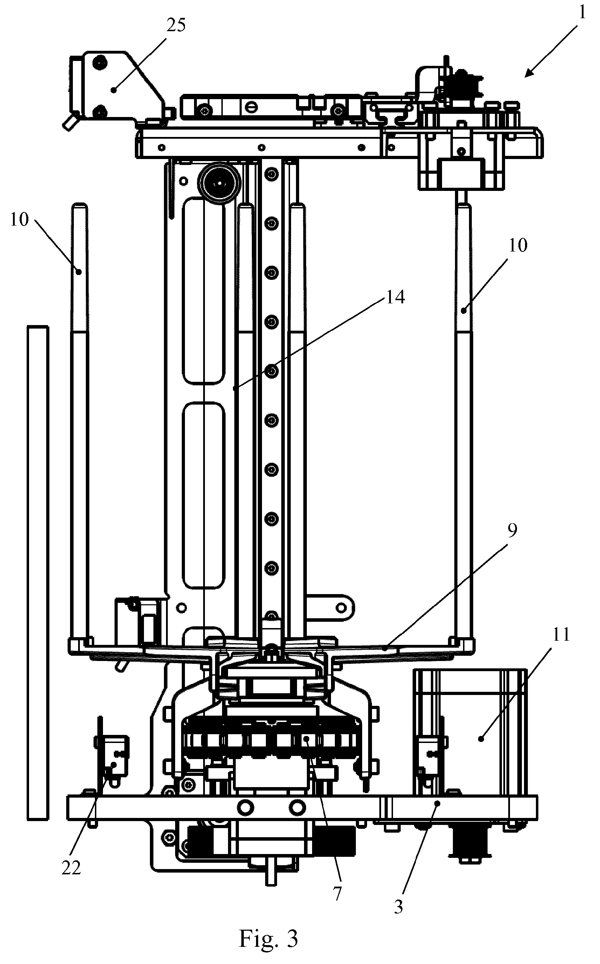

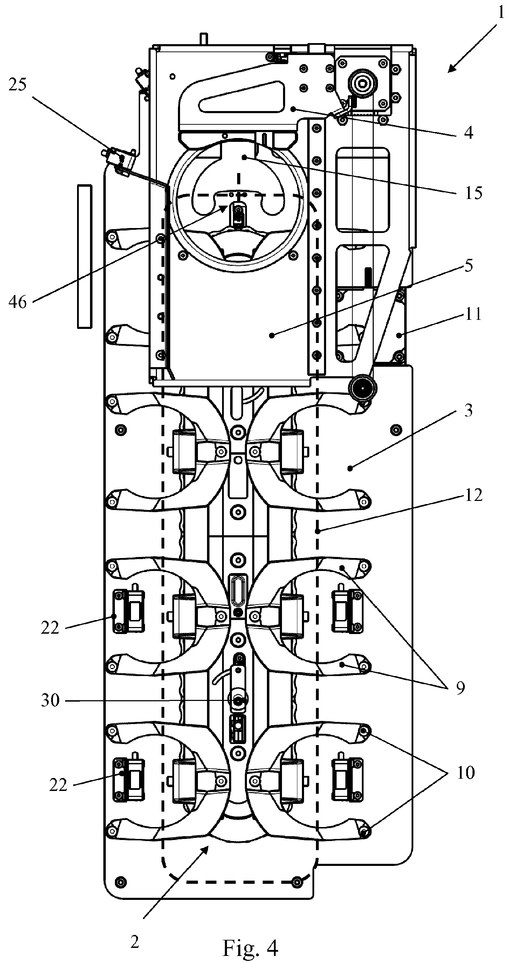

Figures 1, 2, 3 and 4 show an embodiment of an input/output system 1 according to the present invention with an endless conveyor 2, an elevator 13, a pusher 4 displaceably mounted on a conveyor platform 5 and a support 3, wherein the elevator 13 and the endless conveyor 2 are mounted on the support 3. Figure 1 shows the input/output system 1 in a perspective view, figure 2 shows the input/output system 1 in a side view, figure 3 shows the input/output system 1 in a front view and figure 4 shows the input/output system 1 in a top view.

The endless conveyor 2 is of an elongated shape and comprises a chain 7, which chain 7 is guided by a guiding element 8 in a circumferential way. On the chain 7 a plurality of racks 6 are arranged. Each rack 6 comprises a U-shaped base 9 and three poles 10 mounted on the base 9, wherein each rack 6 is configured to store a plurality of petri dishes 16 in a stacked manner. The chain 7 is driven by a first motor 11 mounted at the support 3, wherein the first motor 11 is mechanically connected with the chain 7 by a gearing 48. By driving the chain 7 the racks 6 arranged on the chain 7 are displaced along an orbit path 12, as shown in figure 4.

The elevator 13 is mounted on the support 3 at a hand-over position 46 at the orbit path 12. The elevator 13 comprises a guiding element 14 and a shovel 15, which shovel 15 is shown in figure 4. The shovel 15 is movably mounted along a lifting axis 7 at the guiding element 14. The input/output system 1 further comprises a second motor 23, wherein the second motor 23 is configured for driving the shovel 15 along the lifting axis 17.

The conveyor platform 5 is rigidly mounted at the guiding element 14 and comprises an opening 47. The pusher 4 is displaceably mounted on the conveyor platform 5 in the direction of the arrow 19 and in the opposite direction of the arrow 19, wherein a third motor 18 is mounted at the conveyor platform 5 for driving the pusher 4. The pusher 4 can be displaced between a first position and a second position, wherein by displacing the pusher 4 from the first position to the second position a petri dish 16 located the receiving position 20 is moved in the direction of the arrow 19 to a processing stage connected to the conveyor platform 5, which processing stage is not shown in figures 1 to 4.

Furthermore, the input/output system 1 comprises a first sensor 24 and a second sensor 25, which both are configured as optical sensors. The first sensor 24 is arranged at a bottom position of the elevator 13 monitoring the filling level of the rack 6 positioned at the hand-over position 46. The second sensor 25 is arranged at the conveyor platform 5 monitoring the presence of a petri dish 16 at the receiving position 20.

At a U-turn of the endless conveyor the racks 6 can be aligned in such a manner, that four racks 6 have an equal distance to each other forming a petri dish input section 21. In the middle of the four racks 6 forming the petri dish input section 21 a releasing device 30 is arranged at the endless conveyor 2. Furthermore, the input/output system 1 comprises third sensors 22, which are arranged at the support 3 at the petri dish input section 21. The third sensors 22 are configured to monitor the filling level of the racks 6 at the petri dish input section 21 and are configured as optical sensors.

Moreover, the input/output system 1 comprises a control unit, which control unit is not shown in the figures and is connected to the first sensor, the second sensor, the third sensors, the first motor, the second motor and the third motor. The control unit is configured to control the input/output system.

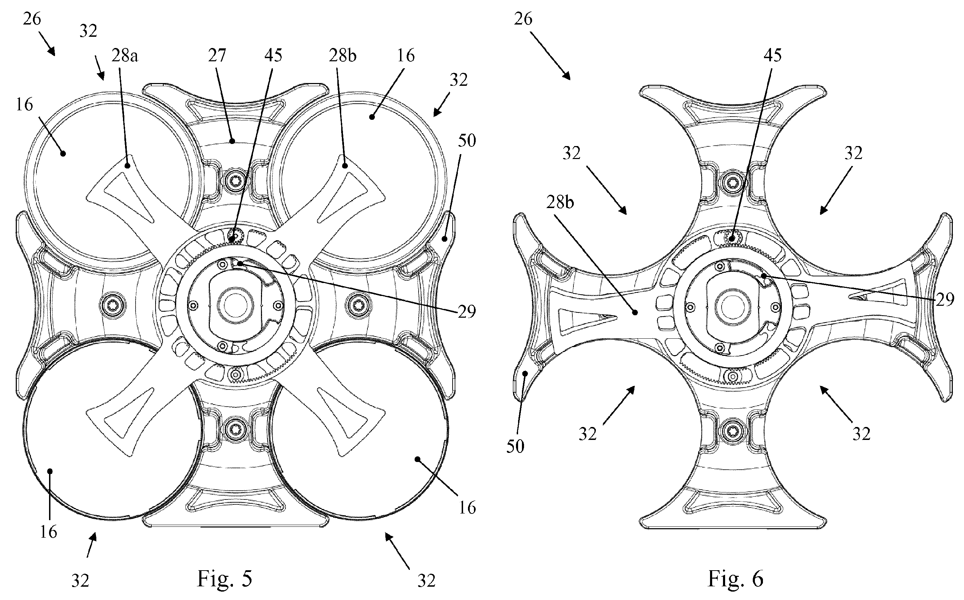

Figures 5 and 6 show an embodiment of the carrier 26 according to the invention from a view from below for supplying petri dishes 16 to an input/output system 1 according to figures 1 to 4. The carrier 26 has a body 27 and a mechanism comprising a first retaining element 28a and a second retaining element 28b, a spring, which spring is not shown, a releasing element configured as an engagement element 29 and one gear wheel 45, which gear wheel 45 mechanically connects the first retaining element 28a with the second retaining element 28b. The retaining elements 28a und 28b are pivotally mounted at the bottom of the body 27 and are displaceable between a locking position and a releasing position, wherein the spring pushes the retaining elements 28a and 28b in the locking position. Please see figure 5. When the carrier 26 is attached to the input/output system 1 the engagement element 29 positively engages with the releasing device 30. By operating the engagement element 29 by means of the releasing device 30, the engagement element 29 is rotated by 45° degrees, whereby also the second retaining element 28b non-rotatably connected to the engagement element 29 is rotated by 45° and thereby displaced into the releasing position. Due to the mechanical connection between the retaining elements 28a and 28b by the gear wheel 45 the first retaining element 28a is rotated in the opposite direction and thereby the first retaining element 28a is also displaced into the releasing position. In the releasing position a locking pin engages with an opening in the second retaining element 28b thereby holding the retaining elements 28a and 28b against the force of the spring in the releasing position. The locking pin and the opening in the second retaining element 28b are not shown. With the retaining elements 28a and 28b located in the releasing position the carrier 26 can be detached from the input/output systems 1 leaving the petri dishes 16 in the racks 6 of the endless conveyor 2. Please see figure 6.

Furthermore the carrier 26 comprises a unlocking button, which unlocking button is configured at the bottom side of the carrier 26 and is not shown. The unlocking button is connected to the locking pin, wherein by putting down the carrier 6 onto a surface or a another processing stage in a petri dishes processing apparatus for loading petri dishes 16 the unlocking button is operated due to the weight of the carrier 26. By operation the unlocking button the locking pin releases the retaining elements 28a and 28b, whereby the retaining elements 28a and 28b are displaced back into the locking position by the spring. The carrier 26 can be loaded again with petri dishes 16.

The body 27 comprises four compartments 32 configured to hold a plurality of petri dishes 16 in a stacked manner. The compartments 32 are partially defined by sidewalls and are open at an upper side of the body 27 and at a bottom of the body 27. Due to the design of the compartments 32 the petri dishes 16 stored in the compartments 32 are positively hold. The body 27 of the carrier 26 is designed in such a manner that the body 27 does not have any inaccessible edges and all edges are rounded. This has the advantage that the carrier 26 is very easy to clean.

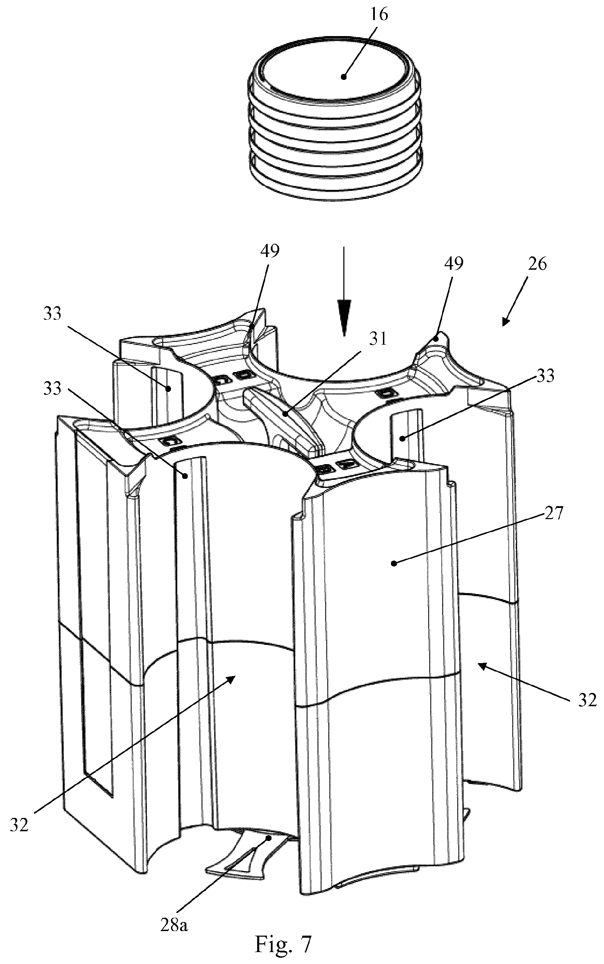

Figure 7 shows the embodiment of the carrier 29 according to figure 5 or 6 in a perspective view. On an upper side of the body 27 a handle 31 is retractably mounted on the body 27 for carrying the carrier 26. In the view shown in figure 7 the handle 31 is in the retracted position. By lifting the carrier 26 the handle 31 is extracted, whereby the carrier 26 is very easy to carry. Each compartment 32 comprises recesses 33 in the sidewalls, which recesses 33 are configured to receive guiding elements arranged at the input/output system 1. The guiding elements are configured by the poles 10 of the racks 6. The poles 10 and racks 6 are not shown in figure 7 for simplicity reasons. The body 27 is made out of Polycarbonate. Furthermore, the body 27 of the carrier 26 comprises on its upper side four male connection pieces 49 and on its bottom four female connection pieces 50 to staple the carriers 26 one above the other. The female connection pieces 50 are shown in figure 5 and 6.

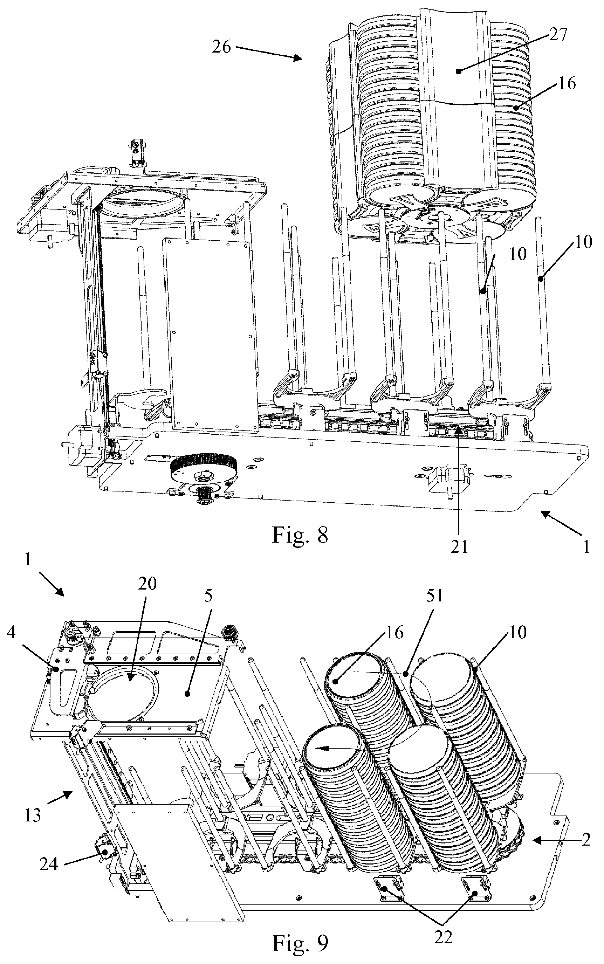

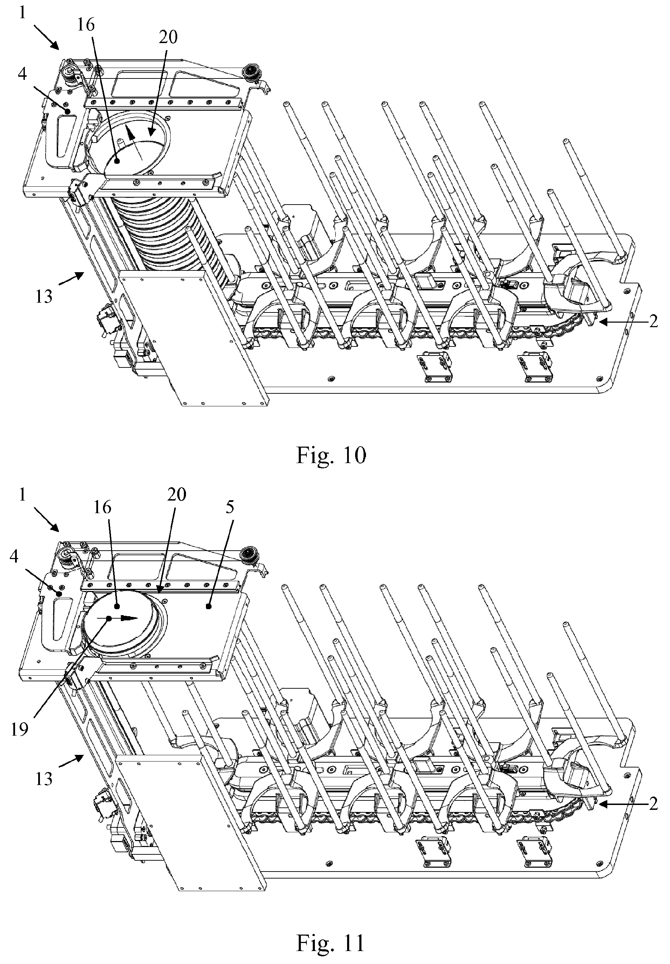

Figures 8 to 11 show the supply of petri dishes 16 to a processing stage in a petri dishes processing apparatus by the input/output system 1 according to figures 1 with a carrier 26 according to figure 5 in a perspective view. The carrier 26 is configured to store eighty petri dishes 16, wherein each compartment 32 is configured to store twenty petri dishes 16. By positioning the carrier 26 above the petri dish input section 21, the poles 10 of the racks 6 situated at the petri dish input section 21 align with the recesses 33 configured in the sidewalls of the compartments 32. By putting the carrier 26 down on the endless conveyor 2, the carrier 26 is guided by the poles 10 in the right direction, whereby each stack 6 of petri dishes 16 stored in the carrier 26 is transferred into a respective rack 6 positioned at the petri dish input section 21. Simultaneously, by putting the carrier 26 down on the endless conveyor 2, the engagement element 29 situated on the bottom of the body 27 of the carrier 26 engages with the releasing device 30 of the input/output system 1. The releasing device 30 displaces the first retaining element 28a and the second retaining element 28b from the locking position to the releasing position, whereby the petri dish 16 situated at the very bottom of each stack rests on the respective base 9 of the individual rack 6. By lifting the carrier 26 with its handle 31, the petri dishes 16 are released onto the endless conveyor 2. See figure 8 and 9. The emptied out carrier 26 can be loaded with another batch of petri dishes 16.

By driving the endless conveyor 2 the stacks are displaced in a circumferential direction 51 along the orbit path 12 one by the other to the hand-over position 46. The stack of petri dishes 16 contained in the rack 6 located at the hand-over position 46 is stepwise lifted by the height of the uppermost petri dish 16 of the stack of petri dishes 16 by means of the elevator 13, such that the uppermost petri dish 16 is presented to the receiving position 20 on the conveyor platform 5. The pusher 4 is configured to forward the petri dishes 16 individually from the receiving position 20 in a direction of the arrow 19 to the processing stage of the petri dishes processing apparatus. After each rack 6 is emptied out or when four empty racks 6 are located at the petri dish input section 21, respectively, the input/output system 1 can be loaded again with a another carrier 26 full or partially full of petri dishes 16. This whole process of transferring the petri dishes 16 from the petri dish input section 21 to the processing stage of the petri dishes processing apparatus is controlled by the control unit by means of the first sensor 24, the second sensor 25, the third sensors 22, the first motor 11, the second motor 23 and the third motor 18.

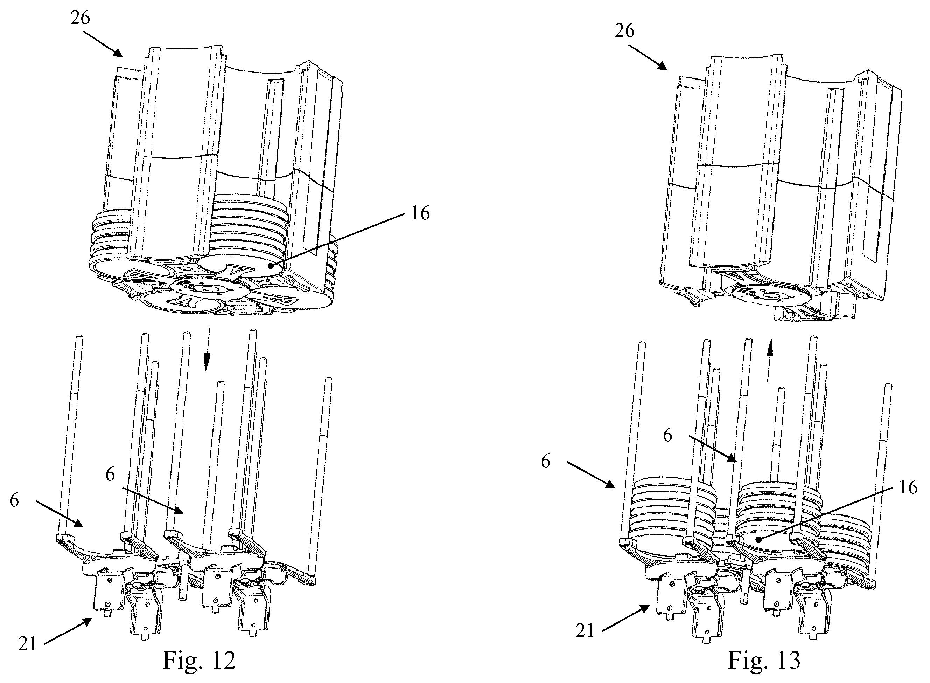

Figures 12 and 13 show the transfer of the petri dishes 16 from the carrier 26 to the racks 6 situated in the petri dish input section 21 in more detail.

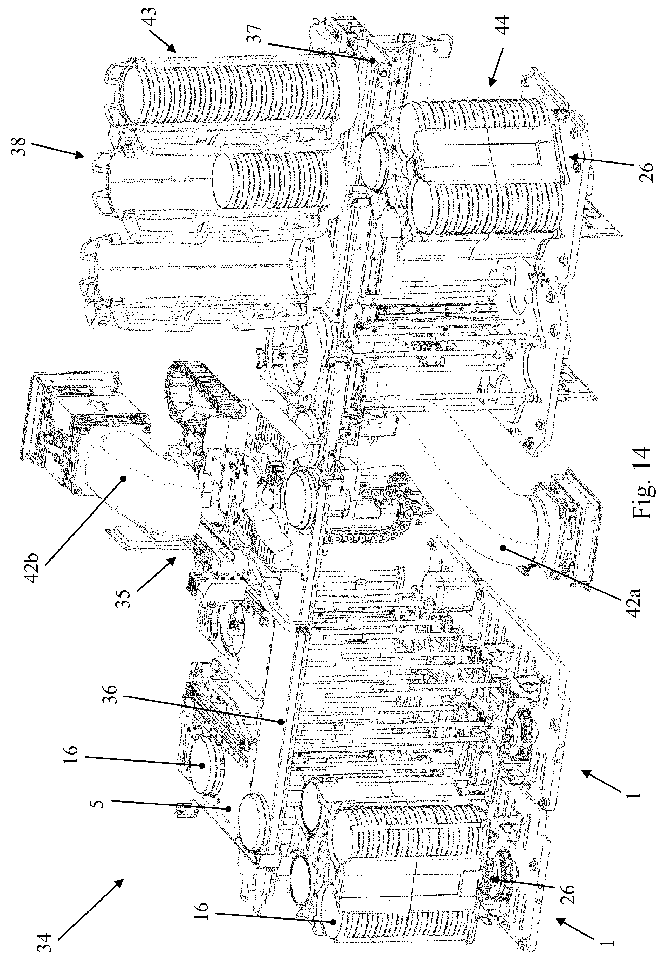

Advantageously, the input/output system 1 according to figures 1 to 4 is integrated in a petri dishes processing apparatus 34 for analyzing, preferably optically analyzing, petri dishes 16, as seen in figure 14. The processing apparatus 34 comprises two input/output systems 1 according to figures 1 for the supply of petri dishes 16, a transfer and handling system 35, a slipway 36, a band conveyor 37 and a system 38 for sorting the petri dishes 16. The input/output systems 1 are arranged next to each other, wherein each input/output systems 1 is connected with the slipway 36 by means of its conveyor platform 5. The slipway 36 connects the input/output systems 1 with the transfer and handling system 35. The petri dishes 16 outputted by the input/output system 1 are forwarded to the transfer and handling system 35 by means of a slider 39.

The transfer and handling system 35 comprises a device for centring of petri dishes 40, a gripper 41 for opening the petri dishes 16 and moving the bottom container of the petri dishes 16 into a visual analysis tool not shown in figure 14. In a further movement the gripper 41 closes the petri dishes 16. To avoid any contamination of the probes extraction systems 42a, 42b are configured to generate a slight vacuum in the area where the petri dishes 16 are opened by the gripper 41.

After an individual petri dish 16 was optically captured a next petri dish 16 pushed by to slider 39 towards the transfer and handling system 35 pushes the petri dish 16 which has just been optically captured to the band conveyor 37. The band conveyor 35 transports the optically captured petri dishes 16 to the system 38 for sorting the petri dishes 16 according to the result of the visual analysis tool. Depending on the result of the visual analysis tool the system 38 for sorting the petri dishes 16 supplies the petri dishes 16 to a first output module 43 or a second output module 44 for further processing.

## Claims

1. An input/output system (1) for automatically supplying petri dishes (16) to a processing stage in a petri dishes processing apparatus (34), comprising:
a plurality of racks (6) displaceably arranged in an orbit path (12), each rack (6) being configured to store a plurality of petri dishes (16) in a vertically stacked manner,
an elevator (13) arranged at a hand-over position (46) at the orbit path (12), wherein the elevator (13) is configured to lift a stack of petri dishes (16) contained in one of said racks (6), while said rack (6) is positioned at the hand-over position (46), **characterized in that** the plurality of racks (6) are arranged on an endless conveyor (2), which endless conveyor (2) is configured to move the racks (6) along the orbit path (12) and to supply one rack (6) after the other to the hand-over position (46),
the elevator (13) is configured to stepwise lift the stack of petri dishes (16) contained in the rack (6) at the hand-over position (46) by the height of the uppermost petri dish (16) of the stack of petri dishes (16) and to repeat said lifting operation for each petri dish (16) of the stack of petri dishes (16), such that the uppermost petri dish (16) is presented to a receiving position (20) on a conveyor platform (5), wherein a pusher (4) is displaceably mounted at the conveyor platform (5) and is configured to receive the petri dish (16) at the receiving position (20) and to move it to the processing stage.

2. An input/output system (1) according to claim 1, **characterized in that** each rack (6) comprises a base (9) for supporting the stack of petri dishes (16) and a plurality of poles (10) mounted on the base (9) and extending essentially parallel to a lifting axis (17) of the elevator (13), wherein the poles (10) are configured to encompass the stack of petri dishes (16).

3. An input/output system (1) according to claim 3, **characterized in that** the endless conveyor (2) is configured as a chain conveyer comprising a chain (7), which chain (7) is driven by a first motor (11), wherein the bases (9) of the racks (6) are mounted on the chain (7).

4. An input/output system (1) according to any of the claims 1 to 3, **characterized in that** the input/output system (1) comprises a first sensor (24) and a second sensor (25), wherein the first sensor (24) is configured to monitor the filling level of petri dishes (16) of the rack (6) while the rack (6) is positioned at the hand-over position (46) and wherein the second sensor (25) is configured to monitor a presence of a petri dish (16) at the receiving position (20) on the conveyer platform (5).

5. An input/output system (1) according to any of the claims 1 to 4, **characterized in that** the endless conveyer (2) comprises a petri dish input section (21), being configured to receive at least one stack of petri dishes (16) and to transfer it into at least one rack (6) positioned at the petri dish input section (21).

6. An input/output system (1) according to claim 5, wherein the petri dish input section (21) is defined by a plurality of the racks (6), preferably by four racks (6), aligned at equal distances from each other, wherein preferably the petri dish input section (21) is located at a U-turn of the endless conveyor (2).

7. An input/output system (1) according to claim 6, **characterized in that** the input/output system (1) comprises third sensors (22) configured to monitor the filling level of racks (6) located at the petri dish input section (21).

8. An input/output system (1) according to claim 6 or 7, **characterized in that** the input/output system (1) comprises a carrier (26), which carrier (26) is configured to load racks (6) located at the petri dish input section (21) with petri dishes (16).

9. A carrier (26) for manually supplying petri dishes (16) to an input/output system (1) of a petri dishes processing apparatus (34), preferably to an input/output system (1) according to any of claims 1 to 8, **characterized in that** the carrier (26) comprises:
a body (27) with at least one compartment (32), preferably with a plurality of compartments (32), configured to hold a plurality of petri dishes (16) in a stacked manner, which at least one compartment (32) is at least partially defined by sidewalls and open at an upper side of the body (27) and at a bottom of the body (27), and
a mechanism for releasing petri dishes (16), which mechanism comprises a releasing element and at least one retaining element (28a. 28b), which at least one retaining element (28a, 28b) is mechanically connected to the releasing element and displaceably, preferably rotatably, mounted at the bottom of the body (27), wherein by operating the releasing element the at least one retaining element (28a, 28b) is displaceable between a locking position and a releasing position, wherein in the locking position the at least one retaining element (28a, 28b) holds the petri dishes (16) inside the compartments (32) and wherein in the releasing position the at least one retaining element (28a, 28b) releases the petri dishes (16) for being discharged through the bottom.

10. A carrier (26) according to claim 9, **characterized in that** the carrier (26) comprises a handle (31), which handle (31) is retractably mounted in or on the body (27) on the upper side of the body (27).

11. A carrier (26) according claim 9 to 10, **characterized in that** the carrier (26) is attachable to the input/output system (1) and that each compartment (32) comprises recesses (33) in its sidewall, which recesses (33) are configured to receive guide elements arranged at the input/output system (1), when the carrier (26) is attached to the input/output system (1).

12. A carrier (26) according to claim 10 or 11, **characterized in that** the handle (31) is the releasing element, wherein by rotating the handle (31) in an opening direction the at least one retaining element (28a, 28b) is displaced from its locking position into its releasing position and wherein by rotating the handle (31) in a locking direction the at least one retaining element (28a, 28b) is displaced from its releasing position into its locking position.

13. A carrier (26) according to claim 12, **characterized in that** the releasing element is configured by at least one engagement element (29), which at least one engagement element (29) is configured for a positive engagement with a releasing device (30) of the input/output system (1), when the carrier (26) is attached to the input/output system (1).

14. A carrier (26) according to claim 13, **characterized in that** the mechanism comprises two retaining elements (28a, 28b) independently pivotally mounted at the bottom of the body (27), wherein one of the two retaining elements (28a) is non-rotatably connected to the engagement element (29), and in addition at least one gear wheel (45), which mechanically connects the two retaining elements (28a, 28b), wherein by operating the engagement element (29) by means of the releasing device (30) the two retaining elements (28a, 28b) are rotated in the opposite direction to each other.

15. A carrier (26) according to any of the claims 9 to 14, **characterized in that** the body (27) is made of Polycarbonate or Acrylnitril-Butadien-Styrol and that the carrier (26) is configured to be stackable with another carrier (26).
